# EUROPÄISCHE PATENTSCHRIFT

(11) **EP 1 349 543 B1**
(45) Veröffentlichungstag und Bekanntmachung des Hinweises auf die Patenterteilung: **15.09.2004**
(21) Anmeldenummer: 02760103.8
(22) Anmeldetag: 15.07.2002
(51) Int. Cl.: A61K 9/20, A61K 35/78

(54) **VERFAHREN ZUR HERSTELLUNG VON TABLETTEN AUS PFLANZLICHEN EXTRAKTEN**
METHOD FOR PRODUCING TABLETS FROM PLANT EXTRACTS
PROCEDE DE PRODUCTION DE COMPRIMES A PARTIR D'EXTRAITS VEGETAUX

(30) Priorität: 03.09.2001 DE 10144108
(43) Veröffentlichungstag der Anmeldung: 08.10.2003
(73) Patentinhaber: STEIGERWALD ARZNEIMITTELWERK GMBH, D-64295 Darmstadt (DE)
(72) Erfinder: KROLL, Ulrike, 64839 Münster (DE); KUPER, Willi, 68649 Gross-Rohrheim (DE)
(74) Vertreter: Wablat, Wolfgang, Dr.Dr.
(86) Internationale Anmeldenummer: PCT/DE2002/002634
(87) Internationale Veröffentlichungsnummer: WO 2003/026620

(56) Entgegenhaltungen:
- EP-A- 0 530 833
- WO-A-00/30605
- WO-A-99/32090

## Beschreibung

Die Erfindung betrifft ein Verfahren zur Herstellung von Tabletten aus pflanzlichen Extrakten, insbesondere Johanniskraut-Extrakt, bei dem ein mit Hilfsstoffen vermischter Trockenextrakt verdichtet und granuliert wird und die mit Feuchteschutzmitteln, Oberflächenglättungsmitteln und Trennmitteln maskierten Extraktkompaktat-Partikel mit weiteren Hilfsstoffen zu Tabletten verpreßt werden.

Arzneimittel in Tablettenform aus pflanzlichen Extrakten zeichnen sich gegenüber der flüssigen Verabreichungsform durch eine zielgerichtetere Freisetzung und genauere Dosierung der arzneilich wirksamen Bestandteile aus und ermöglichen dadurch eine besser zu steuernde Therapie.

Die bisher bekannten festen Darreichungsformen aus pflanzlichen Extrakten sind aber insofern notwendig, als für den Herstellungsprozeß als auch für einen schnellen Zerfall der Tablette und eine schnelle Freisetzung der Wirkstoffe ein hoher Hilfsstoffanteil erforderlich ist, aufgrund dessen und des unter dem Aspekt der Einnehmbarkeit der Tablette begrenzten Tablettenvolumens der Wirkstoffanteil in einer Tablette vergleichsweise gering ist.

Mit dem aus der DE 199 57 472 A1 bekannten, eingangs erwähnten Verfahren zur Herstellung von Tabletten mit einem Johanniskrautextrakt gelingt es, eine Tablette in oral einnehmbarer Größe mit einem Extraktanteil von etwa 65 % oder etwa 600 mg herzustellen, und zwar im wesentlichen dadurch, daß in der Größe homogene Partikel, die aus einem unter Verwendung eines relativ hohen Hilfsstoffanteils hergestellten Extraktkompaktat gebildet sind, mit Titandioxid, Talkum und hochdispersem Siliziumdioxid überzogen werden. Durch die Granulatbeschichtung mit den zuletzt genannten Hilfsstoffen wird zum einen der für ein gutes Fließverhalten und das homogene Mischen mit weiteren Hilfsstoffen erforderliche Feuchteschutz sowie eine glatte Oberfläche der Kompaktatpartikel erreicht, um den Anteil der weiteren Hilfsstoffe beim Mischen und Beschichten gering zu halten und in einer einzigen Tablette einen hohen Extraktanteil unterbringen zu können. Eine weitere wünschenswerte Erhöhung des Extraktanteils ist bei der Tablettenherstellung mit Entmischungsproblemen, feuchtempfindlichen oder zu festen Kernen oder Schwierigkeiten beim Vorpressen verbunden. Zudem bereitet bei einem weiter erhöhten Extraktanteil auch die Einhaltung der vorgeschriebenen Zeiten für das Zerfallen der Tablette und die Freisetzung der Wirkstoffe im Körper Schwierigkeiten.

Das Kernproblem bei der Herstellung von Tabletten mit einem möglichst hohen Extraktanteil und dementsprechend geringen Hilfsstoffanteil besteht mithin darin, einerseits eine fehlerfreie Tablettierung und andererseits eine schnelle Freisetzung der arzneilichen Wirkstoffe zu gewährleisten.

Der Erfindung liegt die Aufgabe zugrunde, ein Verfahren anzugeben, das unter Vermeidung der durch Feuchtigkeit, Entmischung oder Zerfall bedingten Tablettierungsprobleme die Herstellung einer aus pflanzlichen Extrakten gebildeten Tablette mit gegenüber den bekannten festen Darreichungsformen dieser Art deutlich erhöhtem Extraktanteil und guten Zerfalls- und Freisetzungseigenschaften ermöglicht.

Erfindungsgemäß wird die Aufgabe mit einem durch die Merkmale des Patentanspruchs 1 gekennzeichneten Verfahren gelöst.

Weitere Merkmale und konkrete Ausgestaltungen der Erfindung sind in den Unteransprüchen angegeben.

Der grundlegende Erfindungsgedanke besteht nach einem ersten Merkmal zunächst darin, daß die Kompaktierung, das heißt, das Verpressen des vorzugsweise auf einem Bandtrockner getrockneten Extrakts mit dem auch als Trennmittel wirkenden hochdispersen Siliziumdioxid durchgeführt wird. Gemäß einem zweiten Erfindungsmerkmal werden Extraktkompaktat-Partikel mit einer gegenüber dem bekannten Verfahren durch den Einsatz gröberer Partikel insgesamt reduzierten Oberfläche eingesetzt, um den für die Maskierung der Extraktkompaktat-Partikel zum Feuchtigkeitsschtz sowie zur Verbesserung des Preßverhaltens und der Zerfallseigenschaften benötigten Hilfsstoffanteil gering halten zu können. Zur Oberflächenreduzierung der Gesamtheit der Extraktkompaktat-Partikel wird eine vorgegebene Korngrößenverteilung eingestellt, indem nach der Siebklassierung nur bestimmte Korngrößen in bestimmten Mengen für die Maskierung und das anschließende Tablettieren verwendet werden. Um bei dem geringen Hilfsstoffanteil dennoch den Anforderungen an einen schnellen Zerfall einer mit pflanzlichen Extrakten derart hochdosierten fertigen Tablette gerecht zu werden, wird nach einem weiteren Erfindungsmerkmal eine Hilfsstoff-Kombination aus drei Sprengmitteln zudosiert, von denen eines aus Natriumhydrogencarbonat besteht und den Anteil an den beiden anderen Sprengmitteln um ein Mehrfaches übersteigt.

Das Wesen der Erfindung liegt somit in der Kombination aus einer prozentual geringen Zudosierung von hochdispersem Siliziumdioxid bereits vor der Kompaktierung, der Oberflächenreduzierung der Gesamtheit der Extrakt-Kompaktat-Partikel durch eine definierte Korngrößenverteilung und der Verwendung von Natriumhydrogencarbonat in Verbindung mit zwei weiteren, aber signifikant geringer dosierten Sprengmitteln.

Aufgrund dieser Merkmalskombination gelingt es, eine in der Größe akzeptable Tablette mit geringem Hilfsstoffanteil und einem hohen Extraktanteil von etwa 80 % herzustellen, die ohne Tablettierungsprobleme hergestellt werden kann, im Körper ausreichend schnell zerfällt und den Anforderungen an eine schnelle Freisetzung der arzneilichen Wirkstoffe genügt.

In Ausgestaltung der Erfindung erfolgt das Trocknen des eingeengten Extrakts auf einem Bandtrockner unter Vakuum. Der Anteil an dem bereits dem Trockenextrakt beigemischten Siliziumdioxid beträgt lediglich 1 %. Zum Feuchteschutz und zur Oberflächenglättung werden die Extraktkompaktat-Partikel in bekannter Weise mit Titandioxid und Talkum maskiert.

Gemäß einem weiteren wichtigen Erfindungsmerkmal wird zur Erzielung einer gegenüber dem bekannten Verfahren verringerten Oberfläche, die in Kombination mit den erfindungsgemäß eingesetzten Sprengmitteln das angestrebte Zerfallen der mit pflanzlichen Wirkstoffen hochdosierten Tablette gewährleisten soll, eine Korngrößenverteilung der Extraktkompaktat-Partikel eingestellt, wonach mindestens 99 % kleiner als 1000 µm, mindestens 90 % kleiner als 710 µm, 40 - 80 % kleiner als 500 µm, 30 - 60 % kleiner als 250 µm und maximal 40 % kleiner als 125 µm sind. Mit dieser Korngrößenverteilung wird insgesamt eine Partikeloberfläche vorgegeben, durch die der Anteil an Hilfsstoffen, mit denen die Partikel ummantelt werden, weiter eingeschränkt werden kann.

Nach einem weiteren Merkmal der Erfindung besteht die Sprengmittelkombination bei der vorgegebenen Korngrößenverteilung/Oberflächengröße der Extraktkompaktat-Partikel zu 5 bis 15 % aus Natriumhydrogencarbonat und jeweils zu 0,5 bis 5 % aus Croscarmellose-Natrium und Carboxymethylstärke-Natrium. In Kombination mit Natriumhydrogencarbonat können in der genannten Konzentration auch andere gängige Sprengmittel, wie zum Beispiel Stärkederivate, Celluloseverbindungen oder Polyvinylpyrrolidon, verwendet werden. Die gewünschten Zerfallszeiten werden jedoch nur durch den synergistischen Effekt der in der beanspruchten Kombination eingesetzten, aber in ihrem Anteil an der gesamten Hilfsstoffmenge begrenzten Sprengmittel erreicht. Erfindungsgemäß können auch die Schritte der Trockenkompaktierung oder der Wirbelschichtgranulierung eingesetzt werden.

Ein die Erfindung beispielhaft wiedergebendes Verfahren zur Herstellung von Filmtabletten mit Johanniskraut als wirksamem Bestandteil wird nachfolgend näher beschrieben.

Ein aus Knospen und Blüten von Johanniskraut unter Verwendung eines Ethanol-Wasser-Gemisches erzeugter Flüssigextrakt wird nach der Filtration durch Trocknung bei 40 bis 60° C unter reduziertem Druck auf ca. 60 % eingeengt. Nach der anschließenden Hochtemperaturbehandlung (15 Sekunden bei 140° C) zur Keimreduzierung wird der eingeengte Extrakt einer schonenden Bandtrocknung bei Temperaturen zwischen 30 und 60° C und bei verringertem Druck unterzogen.

In dem darauffolgenden Schritt wird dem Johanniskraut-Extrakt als Hilfsstoff für die anschließende Kompaktierung hochdisperses Siliziumdioxid in der Größenordnung von 1 % zudosiert, wobei die Extraktpartikel in einem Mischvorgang mit dem Siliziumdioxid ummantelt werden. Schließlich wird der so vorbereitete Extrakt zunächst mechanisch zu einem Kompaktat zusammengepreßt und danach in einem Trommelmischer zerkleinert. Das Gemisch aus verdichteten Extraktkompaktat-Partikeln wird nun gesiebt, wobei für die Weiterverarbeitung zu Tabletten zur Erzielung einer insgesamt verringerten Oberfläche gröbere Partikel verwendet werden. Die Korngrößenverteilung wird so eingestellt, daß mindestens 99 % kleiner als 1000 µm, mindestens 90 % kleiner als 710 µm, 40 bis 80 % kleiner als 500 µm, 30 bis 50 % kleiner als 250 µm und max 40 % kleiner als 125 µm sind, woraus sich eine Schüttdichte der für die Weiterverarbeitung vorgesehenen Extraktpartikel von 0,63 bis 0,80 g/ml und eine Stampfdichte von 0,73 bis 0,85 g/ml ergibt.

Die Extraktkompaktat-Partikel in der vorgegebenen Korngrößenverteilung werden mit Titandioxid, Talkum und pflanzlichem Magnesiumstearat maskiert und zusätzlich mit einer Sprengmittelkombination, die aus Natriumhydrogencarbonat, Croscarmellose-Natrium und Carboxymethylstärke-Natrium besteht, ummantelt. Anschließend werden die mit den angegebenen Hilfsstoffen versetzten Extraktkompaktat-Partikel in einer Tablettenpresse zu Tablettenkernen mit einer Gesamtmasse von 1090 mg verpreßt, in denen der Anteil an Johanniskrautextrakt 900 mg beträgt, und zwar bei einer einnehmbaren Tablettengröße von 9,5 mm Breite, 20,5 mm Länge und 6,6 mm Höhe. Die Tablettenkerne werden danach noch mit einem Vorlack und einem Farblack überzogen.

## Patentansprüche

1. Verfahren zur Herstellung von Tabletten aus pflanzlichen Extrakten, insbesondere Johanniskraut-Extrakt, bei dem ein mit Hilfsstoff vermischter Trockenextrakt verdichtet und granuliert wird und die mit Feuchteschutzmitteln, Oberflächenglättungsmitteln und Trennmitteln maskierten Extraktkompaktat-Partikel mit weiteren Hilfsstoffen zu Tabletten verpreßt werden,
**dadurch gekennzeichnet, daß** die Kompaktierung allein mit dem als Trennmittel wirkenden hochdispersen Siliziumdioxid durchgeführt wird und für die Weiterverarbeitung Extraktkompaktat-Partikel in einer definierten abgestuften Korngröße und Korngrößenverteilung zur Erzielung einer insgesamt kleinen Kompaktat-Oberfläche verwendet werden, die zusätzlich mit einer Sprengmittelkombination aus überwiegend Natriumhydrogencarbonat und zwei weiteren, geringer dosierten Sprengmitteln ummantelt werden.

2. Verfahren nach Anspruch 1, **dadurch gekennzeichnet, daß** der Anteil des vor der Kompaktierung zudosierten Siliziumdioxids bei 1 % liegt.

3. Verfahren nach Anspruch 1 und 2, **dadurch gekennzeichnet, daß** die Korngröße der für die Weiterverarbeitung bestimmten Extraktkompaktat-Partikel etwa zwischen 125 und 1000 µm liegt.

4. Verfahren nach Anspruch 1, **dadurch gekennzeichnet, daß** eine Korngrößenverteilung gewählt wird, bei der mindestens 99 % < 1000 µm, mindestens 90 % < 710 µm, 40 bis 80 % < 500 µm, 30 bis 60 % < 250 µm und maximal 40 % < 125 µm sind.

5. Verfahren nach Anspruch 4, **dadurch gekennzeichnet, daß** die aufgrund der Korngrößenverteilung erzielbare Schüttdichte der Extraktkompaktat-Partikel bei 0.63 bis 0,80 g/ml und die Stampfdichte bei 0,73 bis 0,85 g/ml liegt.

6. Verfahren nach Anspruch 1, **dadurch gekennzeichnet, daß** der Anteil an dem Sprengmittel Natriumhydrogencarbonat zwischen 5 und 15 % und der beiden mit diesem kombinierten weiteren Sprengmittel jeweils zwischen 0,5 und 5 % liegt.

7. Verfahren nach Anspruch 6, **dadurch gekennzeichnet, daß** als weitere Sprengmittel Croscarmellose-Natrium und Carboxymethylstärke-Natrium in Kombination mit dem Natriumhydrogencarbonat zugemischt werden.

8. Verfahren nach Anspruch 6, **dadurch gekennzeichnet, daß** in Kombination mit Natriumhydrogencarbonat als weitere Sprengmittel Stärkederivate, Celluloseverbindungen oder Polyvinylpyrrolidone verwendet werden.

9. Verfahren nach einem der Ansprüche 1 bis 8, **dadurch gekennzeichnet, daß** den Extraktkompaktat-Partikeln als weiterer Hilfsstoff pflanzliches Magnesiumstearat zugemischt wird.

10. Verfahren nach Anspruch 1, **dadurch gekennzeichnet, daß** das Trocknen des eingeengten Extrakts im Rahmen einer Vakuum-Bandtrocknung durchgeführt wird.

11. Verfahren nach Anspruch 1, **dadurch gekennzeichnet, daß** dem Partikelgemisch als maskierende Hilfsstoffe zur Oberflächenglättung und zum Feuchtigkeitsschutz Talkum und Titandioxid zudosiert werden.

## Claims

1. A method for producing tablets from plant extracts, in particular, St. John's wort extract, in which a dry extract mixed with adjuvant is compacted and granulated and the extract compactate particles are masked with moisture protecting, surface smoothing, and separating agents are pressed into tablets together with other adjuvants, **characterized in that** the compacting step is carried out using only highly dispersed silicon dioxide that acts as a separating agent, **in that** extract compactate particles are selected for further processing in defined stepped particle sizes and a particle size distribution aimed at achieving a small overall granulate surface area, and **in that** the particles are coated with an additional combination of blasting agents consisting mainly of sodium hydrogencarbonate and two other lower-dosed blasting agents.

2. The method according to claim 1, **characterized in that** the percentage of silicon dioxide added prior to compacting is 1%.

3. The method according to claims 1 and 2, **characterized in that** the particle size of the extract compactate particles selected for further processing is approximately between 125 µm and 1,000 µm.

4. The method according to claim 1, **characterized in that** a particle size distribution is selected where at least 99% < 1,000 µm, at least 90% < 710 µm, 40% to 80% < 500 µm, 30% to 60 % < 250 µm and a maximum of 40% < 125 µm.

5. The method according to claim 4, **characterized in that** the bulk density of the extract compactate particles that can be achieved due to the particle size distribution is in the range from 0.63 to 0.80 g/ml and the compacted bulk density is in the range from 0.73 to 0.85 g/ml.

6. The method according to claim 1, **characterized in that** the percentage of sodium hydrogencarbonate as a blasting agent is between 5% and 15% while the percentage of each of the other two blasting agents combined with it is between 0.5% and 5%.

7. The method according to claim 6, **characterized in that** croscarmellose sodium and sodium carboxymethyl starch are used as other blasting agents in combination with sodium hydrogencarbonate.

8. The method according to claim 6, **characterized in that** starch derivatives, cellulose compounds, or polyvinyl pyrrolidones are used as other blasting agents in combination with sodium hydrogencarbonate.

9. The method according to any one of claims 1 through 8, **characterized in that** vegetable magnesium stearate is added to the extract compactate particles as another adjuvant.

10. The method according to claim 1, **characterized in that** the reduced extract is dried in a vacuum conveyor drying process.

11. The method according to claim 1, **characterized in that** talc and titanium dioxide are added as masking adjuvants to the particle mixture for surface smoothing and protection against moisture.

## Revendications

1. Procédé de fabrication de comprimés à partir d'extraits végétaux, en partir d'extrait de millepertuis, selon lequel un extrait sec mélangé à un adjuvant est comprimé et granulé et les particules d'extrait compact masquées par des agents protecteurs d'humidité, des agents de lissage de surface et des agents séparateurs sont pressées avec d'autres adjuvants pour former des comprimés, **caractérisé en ce que** le compactage est à lui seul effectué avec du dioxyde de silicium à forte dispersion agissant à titre d'agent séparateur, et **en ce que** pour le traitement ultérieur, on utilise des particules d'extrait compact dans une grosseur de grains et une répartition granulométrique définies et graduées pour obtenir une surface de produit compacté petite dans l'ensemble qui est additionnellement enrobée par une combinaison d'agents de désintégration constitués en majeure partie par du bicarbonate de sodium et deux autres agents de désintégration à dosage plus faible.

2. Procédé selon la revendication 1, **caractérisé en ce que** la part de dioxyde de silicium additionnellement dosé avant le compactage est de l'ordre d'environ 1 %.

3. Procédé selon les revendications 1 et 2, **caractérisé en ce que** la grosseur de grains des particules d'extrait compact destiné au traitement ultérieur est approximativement entre 125 et 1000 µm.

4. Procédé selon la revendication 1, **caractérisé en ce que** l'on choisit une répartition granulométrique dans laquelle au moins 99 % sont inférieurs à 1000 µm, au moins 90 % sont inférieurs à 710 µm, 40 à 80 % sont inférieurs à 500 µm, 30 à 60 % sont inférieurs à 250 µm et au maximum 40 % sont inférieurs à 125 µm.

5. Procédé selon la revendication 4, **caractérisé en ce que** la densité en vrac des particules d'extrait compact, que l'on peut obtenir en raison de la répartition granulométrique, est de 0,63 à 0,80 g/ml et la densité tassée est de 0,73 à 0,85 g/ml.

6. Procédé selon la revendication 1, **caractérisé en ce que** la part d'agent de désintégration constitué par le bicarbonate de sodium est entre 5 et 15 % et la part des deux autres agents de désintégration combinés à celui-ci est pour chacun entre 0,5 et 5 %.

7. Procédé selon la revendication 6, **caractérisé en ce qu'**à titre d'autres agents de désintégration, on mélange de la croscarmellose sodique et du carboxyméthylamidon sodique en combinaison avec du bicarbonate de sodium.

8. Procédé selon la revendication 6, **caractérisé en ce que** l'on utilise à titre d'autres agents de désintégration des dérivés d'amidon, des composés de cellulose ou de la polyvinylpyrrolidone en combinaison avec du bicarbonate de sodium.

9. Procédé selon l'une des revendications 1 à 8, **caractérisé en ce qu'**aux particules d'extrait compact, on mélange à titre d'autre adjuvant, du stéarate de magnésium végétal.

10. Procédé selon la revendication 1, **caractérisé en ce que** la dessiccation de l'extrait concentré est effectuée dans le cadre d'une dessiccation sous vide sur bande transporteuse.

11. Procédé selon la revendication 1, **caractérisé en ce qu'**on ajoute au mélange de particules du talc et du dioxyde de titane à titre d'adjuvant de masquage pour obtenir un lissage de surface et une protection anti-humidité.
